(19) **Europäisches Patentamt European Patent Office Office européen des brevets**

(11) **EP 4 448 353 B1**

(12) **FASCICULE DE BREVET EUROPEEN**

(45) Date de publication et mention de la délivrance du brevet:
**29.04.2026   Bulletin 2026/18**

(21) Numéro de dépôt: **22835752.1**

(22) Date de dépôt: **13.12.2022**

(51) Classification Internationale des Brevets (IPC):
**B60T 8/172** *(2006.01)*      **B60W 40/06** *(2012.01)*

(52) Classification Coopérative des Brevets (CPC):
**B60W 40/06; B60T 8/1725;** B60W 2300/15;
B60W 2520/10; B60W 2530/20; B60W 2556/50

(86) Numéro de dépôt international:
**PCT/EP2022/085658**

(87) Numéro de publication internationale:
**WO 2023/110894 (22.06.2023 Gazette 2023/25)**

(54) **PROCEDE DE DETERMINATION DE LA PROPRIETE MECANIQUE D'UN SOL AGRAIRE**

VERFAHREN ZUR BESTIMMUNG EINER MECHANISCHEN EIGENSCHAFT EINES LANDWIRTSCHAFTLICHEN BODENS

METHOD FOR DETERMINING A MECHANICAL PROPERTY OF AN AGRICULTURAL SOIL

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorité:   **16.12.2021   FR 2113652**

(43) Date de publication de la demande:
**23.10.2024   Bulletin 2024/43**

(73) Titulaire: **COMPAGNIE GENERALE DES ETABLISSEMENTS MICHELIN**
**63000 Clermont-Ferrand (FR)**

(72) Inventeurs:
- **PATURLE, Antoine**
  **63040 CLERMONT-FERRAND CEDEX 09 (FR)**
- **VAYSSETTES, Jérémy**
  **63040 CLERMONT-FERRAND CEDEX 09 (FR)**
- **PINET, François**
  **63040 CLERMONT-FERRAND CEDEX 09 (FR)**

- **LEFEBVRE, Mickael**
  **63720 Entraigues (FR)**
- **DUPARQUE, Annie**
  **80200 Estrées Mons (FR)**
- **MARTIN, Damian**
  **80200 Estrées Mons (FR)**
- **TOMIS, Vincent**
  **62860 Baralle (FR)**
- **SCHEURER, Olivier**
  **26110 Nyons (FR)**
- **UGARTE, Carolina**
  **60026 Beauvais (FR)**

(74) Mandataire: **M.F.P. Michelin**
**DCJ/PI - F35 - Ladoux**
**23 place des Carmes-Déchaux**
**63040 Clermont-Ferrand Cedex 9 (FR)**

(56) Documents cités:
**FR-A3- 3 088 249     FR-A3- 3 088 427**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

**Domaine de l'invention**

La présente invention concerne la détermination des propriétés mécaniques d'un sol, en particulier son évolution spatiale en temps réel comme la déformabilité de surface. Plus précisément, l'invention propose de déterminer un état de résistance mécanique superficielle du sol et de son évolution à la suite du passage d'un engin agricole au moyen d'un signal de mesure représentatif de la courbure circonférentielle du pneumatique.

**Arrière-plan technologique**

Il s'avère utile en effet de connaître à tout instant les propriétés mécaniques d'un sol, pour interagir avec le conducteur ou avec les systèmes d'assistance à la conduite, de manière à les informer en temps réel de l'évolution des conditions de roulage, et éventuellement de réagir à celles-ci. Notamment, la connaissance d'un état de résistance mécanique du sol permet de régler les conditions d'usage d'un véhicule. Par exemple, en présence d'un sol lâche, la pression de gonflage d'un pneumatique peut être baissée afin d'élargir l'aire de contact entre le pneumatique et le sol, et ainsi de limiter la compaction de celui-ci et l'apparition d'ornière après le passage de l'engin agricole.

Par ailleurs, la détermination locale de la résistance mécanique du sol permet d'évaluer l'influence du pneumatique sur les propriétés mécaniques du sol et identifier les opportunités de mener ou non certaines opérations dépendantes de cet état de résistance mécanique. Par exemple, le passage d'un engin dans une terre trop meuble peut dégrader le sol ou causer l'enlisement de l'engin. Le travail de la terre peut aussi être affecté par l'état de résistance mécanique du sol comme une compaction superficielle de celle-ci.

En couplant des états de résistance mécanique avec des données de géolocalisation synchrones, il est possible d'établir une cartographie de la résistance mécanique du sol d'une parcelle, éventuellement couplée avec d'autres caractéristiques du sol. Une telle cartographie peut s'avérer utilise pour déterminer un aménagement du sol de la parcelle, comme la mise en place d'un drainage du sol ou un décompactage modulé en fonction des besoins du sol.

Enfin le sol, agraire en particulier, est complexe et évolutif en fonction de la profondeur d'observation. Il est caractérisé par des grandeurs physiques comme son humidité, sa texture et sa structure. Généralement l'humidité d'un sol, ou son état hydrique, est défini par la masse relative d'eau qui reste dans le sol une fois sa partie « gravitaire » écoulée. On la nomme humidité massique ou pondérale brute, exprimée en pourcentage et notée H. Cependant la quantité d'eau réellement disponible pour la plante, ou eau capillaire, est plus faible que l'humidité massique car la plante doit fournir un effort de succion pour assimiler cette eau, que l'on nomme potentiel hydrique, exprimé en bar. Classiquement, le potentiel hydrique d'une plante est limité à 16 bars, au-delà l'eau n'est pas disponible pour la plante. A l'inverse, un sol à sa capacité maximale de rétention d'eau demandera un faible effort de succion à la plante, de l'ordre de 0.3 bar : on dit que le sol est à son humidité de « capacité au champ ». Il est judicieux de quantifier l'eau capillaire disponible à un instant ou lieu donné du champ en faisant le rapport entre l'eau pondérable H mesurée et sa valeur maximale à la capacité au champ. On nomme HCC cette eau capillaire « normalisée » par rapport à la capacité au champ, exprimée aussi en pourcentage. Le fait de normaliser par rapport à la capacité au champ permet de définir une humidité indépendante de la texture du sol et représentative de l'eau réellement disponible pour la plante.. La texture du sol correspond à la proportion entre les argiles, les limons et les sables qui le constitue. Ces trois composants se distinguent par leur granulométrie. Ainsi une valeur de HCC de 99% signifie que l'eau capillaire disponible pour la plante correspond à 99% de la capacité au champ, soit une valeur très proche du maximum possible. Or cette valeur de 99 % pour HCC correspond à une humidité pondérale H de l'ordre de 40% pour un sol de faible granulométrie à forte rétention d'eau capillaire comme un sol argileux mais seulement à une humidité pondérale H de l'ordre de 20% pour sol de forte granulométrie à faible rétention d'eau capillaire, comme un sol sableux.

Enfin la structure du sol qui correspond à l'arrangement tridimensionnel du matériau du sol que l'on peut observer à divers horizons, c'est-à-dire sur différentes profondeurs de sol, est caractérisée classiquement par sa densité apparente notée DA. Ce paramètre peut être impacté par le travail du sol notamment sur les horizons superficiels, c'est-à-dire quelques dizaines de centimètres mais aussi par la succession des passages des machines agricoles dans le champ.

La combinaison de l'humidité, de la texture et de la structure du sol aboutit à un matériau complexe ayant des propriétés mécaniques spécifiques.

La demande de brevet FR3088249A3 dont le numéro d'enregistrement est 1860481 décrit une détermination de la fermeté du sol devant le pneu sur la seule base d'une mesure de l'évolution de la courbure d'un pneumatique circulant sur le dit sol. La fermeté du sol est liée à la vitesse de mise à plat du pneumatique ainsi qu'à la longueur de l'aire de contact. La fermeté du sol correspond à l'analyse du sol sur une profondeur relativement importante, ce qui traduit la portance du sol.

Toutefois, cette méthode ne permet pas de déterminer toutes les caractéristiques physiques ou mécaniques du sol pouvant représenter de l'intérêt pour des applications particulières. En particulier, cette méthode ne permet pas de quantifier l'état de résistance mécanique du sol superficiellement, c'est-à-dire l'analyse de la déformabilité du sol sur une profondeur de quelques dizaines de centimètres. De même cette méthode ne permet pas non plus d'évaluer l'impact du passage du véhicule équipé du pneumatique de mesure sur les caractéristiques mécaniques du sol, notamment superficiellement.

Les objets de l'invention qui vont suivre ont pour objectif de déterminer les propriétés mécaniques du sol et d'évaluer ainsi l'impact du passage du pneumatique sur le sol en temps réel afin d'adapter au besoin les conditions de roulage du pneumatique.

**Description de l'invention**

L'invention porte sur un procédé de détermination de la mécanique d'un sol sur lequel roule un pneumatique monté sur un véhicule, ledit pneumatique étant muni d'un capteur configuré pour acquérir un signal de mesure représentatif de l'évolution de la courbure du pneumatique lors du roulement sur un sol, le procédé comprenant les étapes suivantes :

- acquisition par le capteur d'un signal de mesure représentatif de l'évolution de la courbure du pneumatique au cours du roulement,
- détermination à partir du signal de mesure de données de mesure comprenant :

   a) un premier paramètre représentatif d'une vitesse, préférentiellement angulaire, de mise à plat du pneumatique lors du contact avec le sol au cours d'un tour de roue du pneumatique, et
   b) un deuxième paramètre représentatif d'une vitesse, préférentiellement angulaire, de mise en rond du pneumatique lors du décollement avec le sol au cours d'un tour de roue du pneumatique,

- détermination de la propriété mécanique du sol en fonction du premier paramètre et/ou du deuxième paramètre en utilisant une relation linéaire liant ladite propriété mécanique du sol et le premier paramètre et/ou le deuxième paramètre, la propriété mécanique du ;ol étant compris dans le groupe comprenant la profondeur d'ornière, la résistance mécanique du ;ol à l'entrée de l'aire de contact, la résistance mécanique du sol à la sortie de l'aire de contact, la :ompaction du sol engendré par le pneumatique.

Le procédé permet de déterminer en temps réel les propriétés mécaniques d'un sol, préférentiellement à l'échelle globale du pneumatique comme la profondeur d'ornière mais aussi à une échelle locale come par exemple les facteurs de résistance mécanique du sol sur un profondeur donnée depuis le sol, sur lequel roule un pneumatique monté sur un véhicule, sans considération de pression ou de charge du véhicule, de façon simple, précise et fiable, à partir du seul signal de mesure représentatif de l'évolution de la courbure du pneumatique. La prise en compte de la vitesse angulaire permet de s'affranchir automatiquement de la vitesse de roulage du pneumatique.

Ici, les premier et second paramètres sont liés à des effets locaux de l'aire de contact, c'est-à-dire, le changement de courbure à l'entrée ou la sortie de l'aire de contact en observant la vitesse de ce changement. Ce ne sont pas des paramètres à l'échelle globale de l'aire de contact comme peut être par exemple la longueur de l'aire de contact. Comme ces grandeurs sont locales, leur sensibilité est bien plus grande qu'une grandeur globale à l'échelle de l'aire de contact. De ce fait, leur observation individuelle permet de remonter à des propriétés mécaniques locales du sol sur lequel roule le pneumatique, ce qui n'est pas forcément possible, pour ne pas dire impossible, avec une grandeur globale, comme la longueur de l'aire de contact, qui moyennera naturellement l'effet sur la totalité de l'aire de contact. L'exploitation d'une seule grandeur permettant de centrer l'analyse sur une zone locale de l'aire de contact, c'est-à-dire ici la zone située à l'entrée ou la sortie de l'aire de contact. Tandis que la combinaison des grandeurs locales permet d'estimer des propriétés mécaniques à l'échelle de l'aire de contact par la différenciation des réponses mécaniques du capteur à l'entrée et à la sortie de l'aire de contact.

De préférence, la détermination de la propriété mécanique du sol prend en compte la texture, l'état hydrique du sol ainsi que la profondeur afin d'affiner la qualité d'évaluation de la propriété mécanique du sol.

Ce procédé est avantageusement complété par les caractéristiques suivantes, prises seules ou en une quelconque de leur combinaison techniquement possible :

- lors du roulement, au cours d'un tour de roue, la courbure du pneumatique évolue selon un cycle présentant :

   - une partie hors contact avec le sol,

   - une partie en contact avec le sol, dans lequel le premier paramètre est déterminé à partir d'une partie du signal de mesure correspondant à une transition de la courbure du pneumatique entre la partie hors contact avec le sol et la partie en contact avec le sol, et le deuxième paramètre est déterminé à partir d'une partie du signal de mesure correspondant à une transition de la courbure du pneumatique entre la partie en contact avec le sol et la partie hors contact avec le sol

- lors du roulement, au cours d'un tour de roue, la courbure du pneumatique évolue selon un cycle présentant :

    - une partie hors contact avec le sol se caractérisant sur le signal de mesure par une courbure stable,

    - une partie en contact avec le sol se caractérisant sur le signal de mesure par un pic de variation de courbure de contact,

    - une transition dite transition d'entrée entre la partie hors contact avec le sol et la partie en contact avec le sol, se caractérisant sur le signal de mesure par un pic de variation de courbure d'entrée opposé au pic de variation de courbure de contact,

    - une transition dite transition de sortie entre la partie en contact avec le sol et la partie hors contact avec le sol, se caractérisant sur le signal de mesure par un pic de variation de courbure de sortie opposé au pic de variation de courbure de contact, le premier paramètre étant déterminé par une pente entre le pic de variation de courbure d'entrée et le pic de variation de courbure de contact, le second paramètre étant déterminé

par une pente entre le pic de variation de courbure d'entrée et le pic de variation de courbure de contact,
- la relation linéaire est de la forme :

$$F = a + b \times KS_{i\,ou\,j},$$

ou

$$F = a + b \times KS_i + c \times KS_j$$

avec F un facteur de propriété mécanique, $KS_{i\,ou\,j}$ le premier ou le deuxième paramètre, et a, b, c des coefficients fixes préalablement déterminés,
- la propriété mécanique du sol est déterminée en calculant un facteur de propriété mécanique à partir du premier paramètre et/ou du second paramètre, et en comparant ledit facteur de propriété mécanique à des seuils délimitant des classes de propriété mécanique du sol.
- la propriété mécanique du sol est définie sur une profondeur X de sol inférieure à 40 centimètres, potentiellement inférieure 20 centimètres, voire inférieure à 10 centimètres.
- une étape de localisation du véhicule au cours de l'étape d'acquisition du signal délivrant une position au moins bidimensionnelle du véhicule.

Ici, la profondeur d'ornière et la compaction du sol engendrée par le passage du pneumatique sont considérées comme des grandeurs globales du sol à l'échelle du pneumatique. En revanche, la résistance mécanique du sol à l'entrée de l'aire de contact et la résistance mécanique du sol à la sortie de l'aire de contact, qui correspondent à la déformabilité superficielle du sol, sont des grandeurs locales du sol à l'échelle du pneumatique.L'invention concerne également une cartographie de l'hétérogénéité de propriété mécanique du sol d'une surface où roule un pneumatique monté sur un véhicule, ledit pneumatique étant muni d'un capteur configuré pour acquérir un signal de mesure représentatif de l'évolution de la courbure du pneumatique lors du roulement sur la surface comprenant au moins deux déterminations de la propriété mécanique du sol correspondant à deux positions au moins bidimensionnelles du véhicule obtenues par le procédé selon l'invention.

Selon un exemple ne faisant pas partie de l'invention cette description concerne également un pneumatique comprenant un capteur sensible à l'évolution de la courbure du pneumatique, configuré pour générer un signal de mesure représentatif de l'évolution de la courbure du pneumatique lors du roulement sur un sol, comprenant une partie active et une carte électronique, la partie active étant configurée pour générer le signal de mesure, la carte électronique étant configurée pour déterminer des données de mesure comprenant :

    - a) un premier paramètre représentatif d'une vitesse, préférentiellement angulaire, de mise à plat du pneumatique lors du contact avec le sol au cours d'un tour de roue du pneumatique, et
    - b) un deuxième paramètre représentatif d'une vitesse, préférentiellement angulaire, de mise en rond du pneumatique lors du décollement avec le sol au cours d'un tour de roue du pneumatique, le capteur étant configuré pour transmettre les données de mesure à l'extérieur du pneumatique.

L'invention concerne également une unité de traitement de données configurée pour recevoir des données de mesure dérivées d'un signal de mesure représentatif de l'évolution de la courbure du pneumatique lors du roulement sur un sol, lesdites données de mesure comprenant :

- a) un premier paramètre représentatif d'une vitesse, préférentiellement angulaire, de mise à plat du pneumatique lors du contact avec le sol au cours d'un tour de roue du pneumatique, et
- b) un deuxième paramètre représentatif d'une vitesse, préférentiellement angulaire, de mise en rond du pneumatique lors du décollement avec le sol au cours d'un tour de roue du pneumatique, l'unité de traitement de données étant configurée pour déterminer la propriété mécanique du sol en fonction du premier paramètre et/ou du deuxième paramètre en utilisant une relation linéaire liant ladite propriété mécanique du sol et le premier paramètre et/ou le deuxième paramètre, la propriété mécanique du sol étant compris dans le groupe comprenant la profondeur d'ornière, la résistance mécanique du sol à l'entrée de l'aire de contact, la résistance mécanique du sol à la sortie de l'aire de contact, la compaction du sol engendré par le pneumatique.

Préférentiellement l'unité de traitement de données est configurée pour recevoir la position au moins bidimensionnelle du véhicule associée aux données de mesure.

L'invention concerne également un véhicule comprenant :

- au moins un pneumatique,
- au moins capteur sensible à l'évolution de la courbure du pneumatique, configuré pour générer un signal de mesure représentatif de l'évolution de la courbure du pneumatique lors du roulement sur un sol, préférentiellement le capteur est disposé à l'intérieur du pneumatique,
- une unité de traitement de données configurée pour recevoir des données de mesure dérivées du signal de mesure représentatif de l'évolution de la courbure du pneumatique lors du roulement sur un sol et pour déterminer la propriété mécanique du sol en fonction d'au moins une des données de mesure, les données de mesure comprenant

  a) un premier paramètre représentatif d'une vitesse, préférentiellement angulaire, de mise à plat du pneumatique lors du contact avec le sol au cours d'un tour de roue du pneumatique, et
  b) un deuxième paramètre représentatif d'une vitesse, préférentiellement angulaire, de mise en rond du pneumatique lors du décollement avec le sol au cours d'un tour de roue du pneumatique, le véhicule étant configuré pour mettre en œuvre le procédé selon l'invention.

De préférence, le capteur comprend une partie active et une carte électronique, la partie active étant configurée pour générer le signal de mesure, la carte électronique étant configurée pour déterminer les données de mesure, et dans lequel l'unité de traitement de données est disposée à l'extérieur du pneumatique.

Selon un exemple ne faisant pas partie de l'invention cette description concerne également un produit programme d'ordinateur comprenant des instructions de code de programme pour l'exécution du procédé selon l'invention, lorsque ledit programme est exécuté sur un ordinateur. Le produit programme d'ordinateur peut prendre la forme d'un support non transitoire lisible par un ordinateur stockant des instructions de code pour l'exécution du procédé selon l'invention, lorsque ledit support non transitoire lisible par un ordinateur est lu par un ordinateur.

## Description brève des dessins

L'invention sera mieux comprise à la lecture de la description qui va suivre donnée uniquement à titre d'exemple non limitatif et faite en se référant aux figures annexées dans lesquelles les mêmes numéros de référence désignent partout des parties identiques et dans lesquelles :

- la Fig. 1 illustre de façon schématique un pneumatique monté sur une jante d'un véhicule ;
- la Fig. 2 présente un exemple d'un signal de mesure enregistré par un capteur sensible à la courbure du pneumatique lorsque le pneumatique roule ;

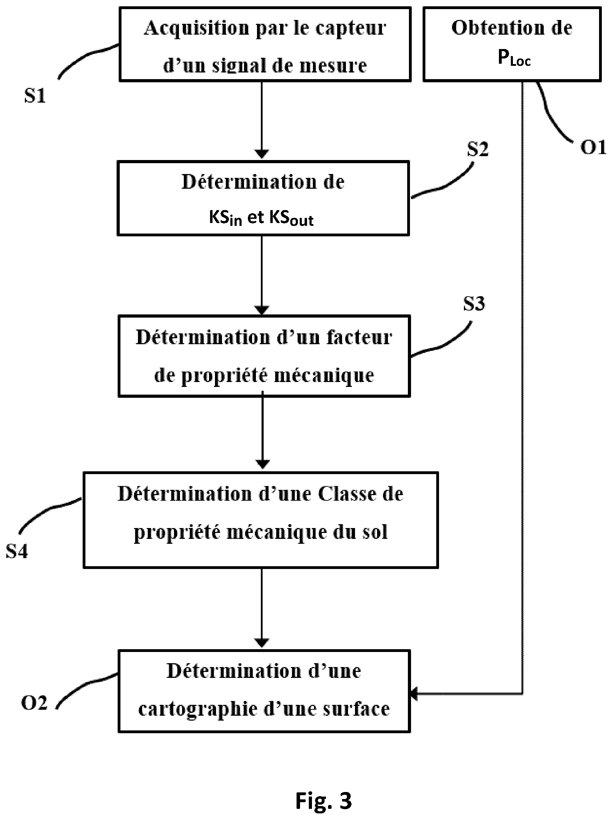

- la Fig. 3 présente un synoptique des étapes de la méthode d'évaluation de la propriété mécanique d'un sol selon des modes de réalisation possibles de l'invention ;
- les Fig. 4a et Fig. 4b montrent chacune un exemple de relation et de classification statistiques entre les deux paramètres dérivés du signal de mesure pour un pneumatique avant d'un véhicule selon différents états de propriétés mécaniques locales du sol correspondants : les résistance mécaniques du sol à l'entrée et la sortie de l'aire de contact ;

- la Fig. 5 montre un exemple de cartographie de l'hétérogénéité d'une propriété mécanique globale du sol, la compaction engendrée par le passage du pneumatique, sur deux parcelles, obtenue à partir du signal de mesure pour un pneumatique de véhicule ;
- la Fig. 6 montre un exemple un exemple de relation de la propriété mécanique globale du sol, la profondeur d'ornière, avec l'un des paramètres dérivés du signal de mesure pour un pneumatique avant d'un véhicule.

## Description détaillée de modes de réalisation

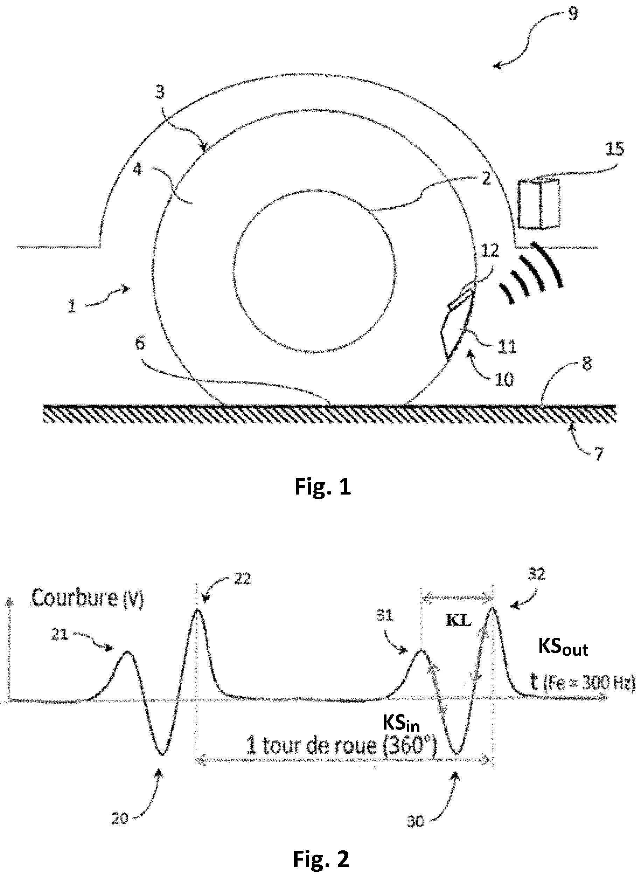

La Fig. 1 illustre un pneumatique 1 monté sur une jante 2. Un tel pneumatique 1 comprend d'une part une zone sommet 3 constituant une bande de roulement présentant des sculptures, et d'autre part des flancs 4 se terminant par des zones basses. Celles-ci comportent généralement une tringle et un talon pour permettre le montage du pneumatique 1 sur la jante 2. La jante 2 est elle-même reliée au véhicule 9 par un essieu (non représenté). Le pneumatique 1 permet ainsi la liaison entre le véhicule 9 et le sol 7.

On entend ainsi par pneumatique un solide souple conçu pour être monté sur la jante 2 d'une roue, généralement sous forme de bandage, afin d'assurer la liaison entre le véhicule 9 et le sol 7, comportant une bande de roulement subissant une modification de son rayon de courbure circonférentielle lorsqu'il est soumis à un effort. Le pneumatique 1 est typiquement formé d'élastomères (par exemple gomme) et éventuellement d'autres matériaux textiles et/ou métalliques. Le pneumatique 1 peut être sans air, et par exemple avec des rayons de polyuréthane flexible qui supportent la bande de roulement. De préférence toutefois, un pneumatique 1 comprend une enveloppe flexible renfermant un intérieur gazeux sous pression, typiquement de l'air. Comme il s'agit du type le plus courant de pneumatique 1, la description qui suit est faite de manière non limitative en référence à un tel pneumatique 1 présentant une pression interne de gaz sous pression.

Le pneumatique 1 est soumis à une force appliquée par le véhicule 9, via l'essieu et la jante 2, en direction du sol 7. Cette force tire son origine de la charge à l'essieu, résultant du poids du véhicule 9. La jante 2 étant indéformable, cette force s'appliquant sur le pneumatique 1 déforme celui-ci lorsque le pneumatique 1 est au contact de la surface 8 du sol 7 : la partie du sommet 3 sous la jante 2 s'aplatit, ce qui augmente la surface de contact 6 du pneumatique 1 avec le sol, tandis que les flancs 4 se gonflent. Cette déformation est d'autant plus prononcée que la pression à l'intérieur du pneumatique est faible et/ou la charge portée par le pneumatique élevée. La nature du sol 7 influe également sur cette déformation, et en particulier l'état mécanique de ce sol 7. En effet, un sol résistant ne se déforme pas ou peu, tandis qu'un sol mou ou lâche se déforme sous l'action du pneumatique 1, de sorte que la déformation du pneumatique 1 est moindre, en partie transférée au sol 7.

La déformation du pneumatique 1 se traduit par une modification de la courbure circonférentielle du pneumatique 1, c'est-à-dire de la courbure de la zone sommet 3. Lorsque le pneumatique 1 roule, cette modification de la courbure parcourt la circonférence du pneumatique 1. Pour un point donné du pneumatique 1, la courbure va donc varier périodiquement à chaque tour de roue.

Le pneumatique 1 est muni d'un capteur 10 configuré pour acquérir un signal de mesure représentatif de l'évolution de la courbure du pneumatique. Ce capteur 10 est disposé à l'intérieur de l'enveloppe du pneumatique 1. De préférence, le capteur 10 est disposé contre la zone sommet 3. Le capteur 10 peut être enfoui dans la structure de l'enveloppe du pneumatique 10, ou bien être rapporté sur celle-ci, et par exemple maintenu en place par une couche adhésive. Le capteur 10 comporte une partie active 11 solidaire de l'enveloppe du pneumatique 1, de sorte que la déformation du pneumatique 1 entraîne une déformation correspondante de la partie active 11 du capteur 10, lequel génère un signal de mesure fonction de la déformation de sa partie active 11. Le signal de mesure est donc bien représentatif de l'évolution de la courbure du pneumatique.

De préférence, le capteur 10 est un capteur piézoélectrique, lequel génère une tension proportionnelle à la variation de flexion. Plus précisément, le capteur 10 peut par exemple comprendre une partie active 11 constituée d'une couche piézoélectrique entre deux couches conductrices. Il est également possible que le capteur 10 soit un capteur résistif, dont l'impédance est proportionnelle à la flexion de la partie active 11 du capteur. On peut également utiliser un accéléromètre, quoique d'utilisation bien plus complexe et demandant un traitement du signal plus important. Le capteur 10 peut également être adapté pour mesurer d'autres paramètres, et en particulier la pression. Le capteur 10 peut être intégré dans un autre équipement électronique installé dans le pneumatique 1, tel qu'un capteur de pression et/ou de température de type TMS, de l'anglais "tyre monitoring system" pour "contrôle automatique du pneu".

Le capteur 10 comprend également une carte électronique 12 reliée à la partie active 11 du capteur 10 et configurée pour recevoir le signal de mesure en provenance de la partie active 11. Cette carte électronique 12 comprend au moins un processeur et une mémoire, et est adaptée pour traiter des données telles que le signal de mesure, pour déterminer des données de mesure à partir du signal de mesure, et pour communiquer ces données de mesure. De préférence, le capteur 10 est associé à un émetteur sans fil, notamment de type radiofréquence, et par exemple du type utilisant la technologie Bluetooth Low Energy (Bluetooth à basse énergie) ou du type appareil de faible puissance opérant dans la bande des 433 MHz (LPD 433), permettant de relayer le signal de mesure à une unité de traitement automatisée de données, de préférence disposée à l'extérieur du pneumatique 1, pour son traitement. L'émetteur sans fil peut faire partie du capteur 10, par exemple en tant que composant de la carte électronique 12, ou être distinct du capteur 10. On peut ainsi par exemple prévoir une antenne à l'intérieur du pneumatique 1. Dans le cas d'une communication sans fil, un récepteur externe peut recevoir les signaux envoyés par les moyens de communication sans fil associés au capteur 10, et les relayer à l'unité de traitement automatisée de données.

Bien entendu, le capteur 10 peut comprendre d'autres éléments permettant son bon fonctionnement, et notamment un module d'alimentation électrique, par exemple constitué par une batterie.

Lorsque le pneumatique 1 roule sur le sol, le capteur 10 acquiert (étape S1) le signal de mesure représentatif de l'évolution de la courbure circonférentielle du pneumatique. Ce signal de mesure peut être directement lié à la courbure (et donc être un signal de mesure de la courbure), et donc en suivre l'évolution, soit être indirectement lié à la courbure. C'est notamment le cas d'un capteur 10 dont la partie active 11 est un capteur piézoélectrique, puisque le signal de mesure correspond alors à la variation de la courbure qui, lorsqu'elle est intégrée, au niveau d'un amplificateur de charge par exemple, devient la courbure. C'est ce type de capteur qui sera utilisé dans les exemples ci-après. Le signal de mesure, généré par la partie active 11 du capteur 10, est ensuite traité par la carte électronique 12 pour déterminer des données de mesure à partir du signal de mesure. Le traitement du signal de mesure vise à extraire les informations utiles dans ce signal, qui sont exploitées par la suite du procédé.

La Fig. 2 montre un exemple schématique d'un signal de mesure enregistré par un capteur 10 sensible à la courbure du pneumatique lorsque le pneumatique 1 roule. Le signal de mesure est ici représenté par sa tension (en V), et désigné par courbure, en fonction de la rotation de la roue exprimée en degré.

Lors du roulement, au cours d'un tour de roue, la courbure du pneumatique évolue selon un cycle présentant :

- une partie hors contact avec le sol,

- une partie en contact avec le sol.

La séquence illustre deux passages dans la partie en contact avec le sol de la zone du pneumatique 1 où est disposé le capteur 10, séparés par une partie de cycle hors contact avec le sol. La partie de cycle hors contact avec le sol se caractérise par une courbure stable, qui se traduit par une stabilité du signal de mesure. La partie du cycle en contact avec le sol se caractérise sur le signal de mesure par un pic de variation de courbure de contact 20, 30. Sur la Fig. 2, les pics de variation de courbure de contact 20, 30 sont dirigés vers le bas. En effet, les pics de variation de courbure de contact 20, 30 correspondent à la mise à plat du pneumatique 1 dans la surface de contact 6., soit une courbure proche de zéro.

La courbure présente également une transition dite transition d'entrée entre la partie hors contact avec le sol et la partie en contact avec le sol, se caractérisant sur le signal de mesure par un pic de variation de courbure d'entrée 21, 31 opposé au pic de variation de courbure de contact 20, 30, c'est-à-dire ici vers le haut. La variation de la courbure présente également une transition dite de sortie entre la partie en contact avec le sol et la partie hors contact avec le sol, se caractérisant sur le signal de mesure par un pic de variation de courbure de sortie 22, 32 opposé au pic de variation de courbure de contact, c'est-à-dire ici vers le haut. Le pic de variation de courbure d'entrée 21, 31 et le pic de variation de courbure de sortie 22, 32 correspondent aux variations plus ou moins brutales de rayon de courbure du pneumatique 1 en entrée et sortie de l'aire de contact.

Puisque le pneumatique tourne, le même cycle se répète, avec un signal de mesure stable hors contact avec le sol, puis un pic de variation de courbure d'entrée 21, 31, un pic de variation de courbure de contact 20, 30, un pic de variation de courbure de sortie 22, 32, et enfin de nouveau un signal de mesure stable hors contact avec le sol. Ce cycle correspond à un tour de roue, donc à 360°, représenté sur la Fig. 2. Pour chaque cycle, le pic de variation de courbure de sortie 22, 32 présente le grand avantage d'être aigu et surtout essentiellement indépendant des conditions de sol et du pneumatique 1. En effet, le pic de variation de courbure de sortie 22, 32 correspond au changement de courbure du pneumatique 1 en sortie de l'aire de contact, quand la zone du pneumatique 1 où est située le capteur 10 passe brutalement de l'état plat caractéristique de la partie en contact avec le sol à l'état courbe caractéristique de la partie hors contact avec le sol. Sur un sol meuble, au fur et à mesure qu'il roule, le pneumatique 1 compacte le sol sous lui, formant une ornière, et donc un fond d'ornière assez ferme sur lequel le pneumatique 1 repose en sortie de l'aire de contact. Par ailleurs, l'avancement du véhicule 9 porte les contraintes essentiellement vers l'entrée de l'aire de contact. Le pneumatique 1 en sortie de l'aire de contact présente ainsi un comportement de sortie, en termes de courbure, très proche du comportement d'un pneumatique 1 sur une route.

Ainsi, il est aisé d'identifier chaque cycle correspondant à un tour de roue, en repérant chaque pic de variation de courbure de sortie 22, 32. Il est également possible d'identifier les cycles avec un dispositif dédié, tel qu'un compte-tour. A partir de là, les données peuvent être exprimées en fonction de degré angulaire de chaque cycle. Cela permet notamment de pouvoir comparer les cycles et leurs données indépendamment de la vitesse du véhicule 9. Les étapes du procédé ne requièrent qu'un cycle pour être mises en œuvre, et peuvent donc être mises en œuvre pour chaque cycle. Toutefois, afin de rendre le procédé plus robuste à d'éventuels aléas ponctuels (présence d'un caillou par exemple), il est possible d'utiliser une combinaison de plusieurs cycles mesurés, par exemple avec une moyenne glissante.

L'état mécanique du sol influence les caractéristiques du profil du signal de mesure. L'invention vise donc à extraire des paramètres du signal de mesure pour en déduire l'état mécanique du sol. Le procédé comprend donc la détermination (étape S2), à partir du signal de mesure de données de mesure comprenant au moins un premier paramètre $KS_{in}$ représentatif d'une vitesse angulaire de mise à plat du pneumatique lors du contact avec le sol lors d'un tour de roue du pneumatique 1, et un deuxième paramètre $KS_{out}$ représentatif d'une vitesse angulaire de mise en rond du pneumatique lors du décollement avec le sol lors d'un tour de roue du pneumatique 1. Les données de mesure peuvent comprendre d'autres paramètres ou valeurs dérivées du signal de mesure.

Les premier $KS_{in}$ et deuxième $KS_{out}$ paramètres sont déterminés à partir d'une partie du signal de mesure correspondant à une transition de la courbure du pneumatique entre la partie hors contact avec le sol et la partie en contact avec le sol. Plus précisément, le premier paramètre $KS_{in}$ est déterminé par une pente entre le pic de variation de courbure d'entrée 31 et le pic de variation de courbure de contact 30. Plus précisément, le premier paramètre $KS_{in}$ peut correspondre à la variation maximale de la courbure entre le pic de variation de courbure d'entrée 31 et le pic de variation de courbure de contact 30, c'est-à-dire correspondre à la pente maximale (en valeur absolue). Dans l'exemple, le signal de mesure s'exprimant en volt V en fonction des degrés angulaires °, le premier paramètre $KS_{in}$ peut avoir pour unité des V/°, c'est-à-dire en dérivée première de la courbure du pneumatique 1.

Le deuxième paramètre $KS_{out}$ est déterminé par une pente entre le pic de variation de courbure de sortie 32 et le pic de variation de courbure de contact 30. Plus précisément, le deuxième paramètre $KS_{out}$ peut correspondre à la variation maximale de la courbure entre le pic de variation de courbure de sortie 32 et le pic de variation de courbure de contact 30, c'est-à-dire correspondre à la pente maximale. Dans l'exemple, le signal de mesure s'exprimant en volt V en fonction des degrés angulaires °, le premier paramètre $KS_{out}$ peut avoir pour unité des V/°, c'est-à-dire en dérivée première de la courbure du pneumatique 1.

Les paramètres $KS_{in}$ et $KS_{out}$ peuvent être approximé de plusieurs façons. Par exemple, les paramètres KS peuvent correspondre au maximum (au sens de la valeur absolue) de la dérivée du signal de mesure entre le pic de variation de courbure d'entrée 31 ou de sortie 32 et le pic de variation de courbure de contact 30, la dérivée étant estimée à partir de la différence entre deux points de mesure successifs (ou proches), en prenant évidemment en compte leur éloignement angulaire. S'agissant dans l'exemple d'une pente décroissante pour le $KS_{in}$, il en sera de même pour le $KS_{out}$ avec une pente croissante, ce maximum au sens de la valeur absolue correspond à un minimum de la dérivée du signal de mesure entre le pic de variation de courbure d'entrée 31 et le pic de variation de courbure de contact 30. Il est également possible, au lieu de rechercher une valeur extrémale de dérivée, de choisir des points de mesure fixes, comme ceux situés à équidistance des sommets du pic de variation de courbure d'entrée 31 et du pic de variation de courbure de contact 30, et de calculer la dérivée à partir de ces points. On peut encore prendre les points de mesure correspondant à une valeur de signal de mesure, comme le passage au « zéro » dans le cas illustré. Il est encore possible d'utiliser des approches plus complexes, comme l'algorithme de Savitzky-Golay. Toutefois, le choix d'une fréquence d'échantillonnage relativement faible, typiquement inférieure ou égale à 500 Hz, et de préférence inférieure ou égale à 400 Hz, comme les 300 Hz de l'exemple, revient à lisser le signal de mesure et permet de choisir des approches moins lourdes en calcul, tel que celles présentées plus haut.

Le premier paramètre $KS_{in}$ et le deuxième paramètre $KS_{out}$ ont l'avantage de présenter une grande variabilité en fonction de la résistance mécanique du sol, et d'être facilement obtenus, comme démontré ci-dessus. Plus précisément, lorsque la résistance du sol diminue, les paramètres $KS_{in}$ et $KS_{out}$ diminuent (en valeur absolue) et inversement. Ainsi, plus le sol est lâche, plus la vitesse de mise à plat ou la vitesse de mise en rond diminue. A l'inverse, lorsque la résistance du sol 7 augmente, les paramètres $KS_{in}$ et $KS_{out}$, ce qui revient à dire les vitesses de déformation du pneumatique, augmentent.

Pris individuellement, les paramètre KS peuvent dépendre de la charge, de la pression, et/ou de la vitesse. Toutefois, la prise en compte à la fois du premier paramètre $KS_{in}$ et du deuxième paramètre $KS_{out}$ permet de déterminer des grandeurs de déformation du sol à l'échelle du pneumatique, comme la compaction sur une profondeur X plutôt superficielle du sol $C_{pneu}$ 0-X engendré par le passage du pneumatique 1, à partir de ces seuls paramètres, sans connaître la charge, la pression et la vitesse du pneumatique 1 sur le sol.

De préférence, c'est la carte électronique 12 du capteur 10 qui détermine à partir du signal de mesure les données de mesure comprenant le premier paramètre $KS_{in}$ et le deuxième paramètre $KS_{out}$. Ces données de mesure sont ensuite transmises par le capteur 10 à une unité de traitement de données 15 qui met en œuvre la suite du procédé. Cette unité de traitement de données 15 est de préférence disposée à l'extérieur du pneumatique 1, par exemple dans le véhicule 9 mais l'unité de traitement 15 peut également être distante du véhicule 9, et la transmission des données peut alors faire intervenir des moyens de transmission intermédiaires. La transmission des données de mesure entre le capteur 10 et l'unité de traitement de données 15 se fait alors de manière sans fil. L'unité de traitement de données 15 comprend typiquement un processeur et une mémoire, et est adaptée pour recevoir et pour traiter les données de mesure lors de la mise en œuvre de la suite du procédé de la détermination des propriétés mécaniques du sol.

Il est possible de transmettre le signal de mesure à l'unité de traitement 15 pour la mise en œuvre de la suite du procédé. Toutefois, la détermination des données de mesure par le capteur 10 et la transmission de ces seules données de mesure vers l'unité de traitement de données 15 présente l'avantage de réduire la quantité de données transmises entre le capteur 10 et l'unité de traitement de données 15. La transmission de données consommant beaucoup d'énergie, transmettre les données de mesure plutôt que le signal de mesure permet de limiter la consommation électrique du capteur 10, dont les possibilités d'alimentation dans le pneumatique 1 sont limitées.

Il est par ailleurs avantageux de ne pas utiliser la carte électronique 12 du capteur 10 pour la mise en œuvre de la suite du procédé, mais plutôt d'utiliser l'unité de traitement de données 15 pour traiter les données de mesure. On limite ainsi les calculs effectués par la carte électronique 12 du capteur 10, ce qui permet d'économiser de l'énergie et de la mémoire au niveau de la carte électronique 12. De plus, il est plus aisé de modifier les modalités de mise en œuvre de la suite du procédé sur une unité de traitement de données 15 facilement accessible, plutôt que sur le capteur 10 à l'intérieur du pneumatique 1.

Une fois que l'unité de traitement 15 a reçu les données de mesure, l'unité de traitement 15 peut déterminer la propriété mécanique du sol en fonction du premier paramètre $KS_{in}$ et du second paramètre $KS_{in}$ contenus dans les données de mesure, qui varient en fonction de la résistance mécanique superficielle du sol, comme montré ci-dessous.

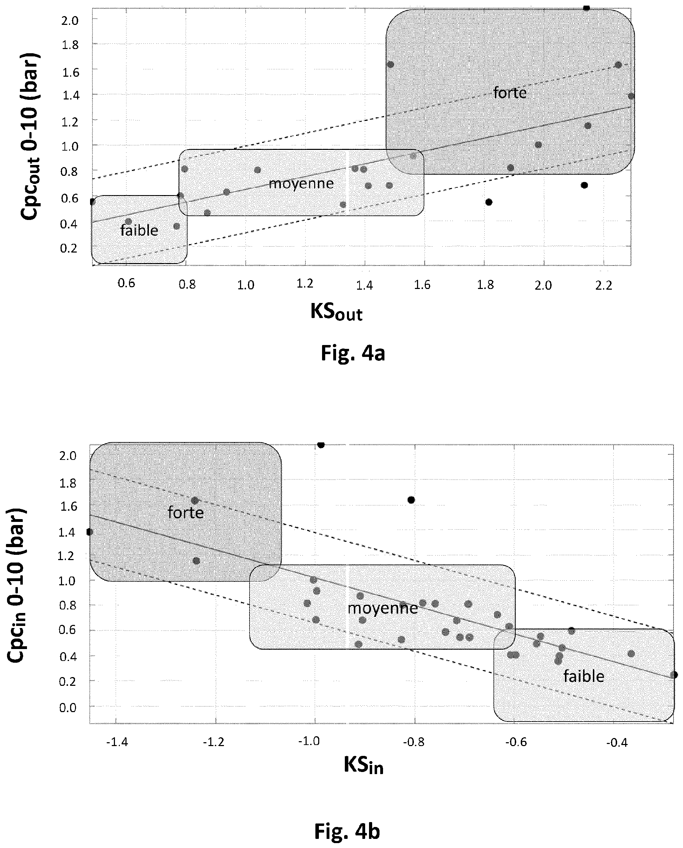

Les Fig. 4a et 4b sont des illustrations de la détermination d'une propriété mécanique du sol qui est locale et non globale à l'échelle du pneumatique.

Dans ces exemples, les données de mesure dérivent d'un signal de mesure acquis par un capteur 10 piézoélectrique disposé dans un pneumatique avant d'une remorque déportée et tirée par un tracteur agricole lors du passage du dit tracteur. Cela permet de disposer sur la remorque des équipements de mesure nécessaires pour déterminer la pression et la charge appliquée au pneumatique de manière stabilisée. Le déport de la remorque, en particulier du pneumatique de mesure par rapport aux pneumatiques du tracteur, assure de ne pas tasser le sol par le passage du tracteur mais bien par le seul passage du pneumatique de mesure.

Dans les résultats des deux figures Fig. 4a et Fig. 4b, le tracteur a roulé sur plusieurs natures de sols représentant trois conditions de textures différentes :

- un sol de type limon moyen,

- un sol de type limon argileux,

- un sol de type sablonneux.

De plus, ces différents sols ont été mis dans tous les états hydriques possibles en faisant varier la proportion d'eau autant en surface qu'en profondeur, soit naturellement en fonction des saisons (hiver, printemps, été, automne), soit par irrigation.

Ces sols ont aussi été préparés selon trois conditions initiales de structure différentes :

- Un état tassé ou rappuyé nommé « WO » correspond à l'état du sol laissé après la récolte de ce fait tassé par le passage à de nombreuses reprises d'un pneumatique chargé et gonflé sans aucun travail du sol après ces passages.

- Un état de sol remanié ou meuble réalisé par un hersage du sol tassé sur une profondeur de 10 ou 30 centimètres nommés respectivement « W10 » et « W30 ».

De plus, d'autres états structuraux du sol ont été créés en faisant évoluer le nombre de passages du tracteur (jusqu'à trois passages) sur chacun de ces états initiaux. Enfin les mesures ont été faites à différentes conditions de charge et de pression appliquées sur le pneumatique représentatives de l'usage en champ d'un pneumatique agricole. Les mesures ont été faites pour des vitesses de roulages variées en dessous d'une vitesse maximale de 20 km/h.

La première propriété mécanique est la résistance du sol devant le pneu noté $Cpc_{in}$ qui correspond à la détermination de la limite de contrainte élastique admissible par le sol exprimé en bar, c'est à-dire la contrainte maximale que le sol peut supporter avant de se déformer de façon irréversible ce qui le conduit à modifier sa structure. Nécessairement, plus cette contrainte est basse plus le sol est peu résistant ou lâche. Inversement plus cette contrainte est élevée, plus le sol est résistant. La résistance maximale s'obtient pour un sol de type route qui ne se déforme pas sous le passage d'un pneumatique agricole en condition normale d'utilisation.

La Fig. 4a montre une relation linéaire entre le $Cpc_{in}$ et la vitesse de mise à plat du pneumatique lors du contact avec le sol $KS_{in}$ et ce quel que soit l'état physique du sol, c'est-à-dire sa texture, sa structure et son état hydrique. Les deux droites en pointillés représentent les intervalles de confiance à 90%.

Il est possible de définir des classes de résistance du sol en particulier trois niveaux différents, ce qui permet de différencier la nature de résistance du sol à l'aide d'une caractérisation de type laboratoire de la résistance du sol obtenue par une mesure de compression uni-axiale d'un échantillon cylindrique de terre prélevé sur une certaine profondeur X. Dans le cas de la Fig. 4a, la mesure de Cpc a été effectuée sur une profondeur de 10 centimètres sur des carottages du sol ayant été prélevés avant ou après le passage du pneumatique.

A titre d'exemple illustratif et non limitatif, les classes suivantes peuvent être utilisées :

[Table 1]

| Cpc (bar) | <= 0.5 bar | 0.5 - 1.0 bar | > 1.0 bar |
|---|---|---|---|
| Résistance du sol | Faible ou « lâche » | Moyenne ou « intermédiaire » | Forte ou « résistant » |
| $KS_{in}$ | >= -0.6 | -1.1 - -0.6 | < -1.1 |

Il a été identifié un niveau de corrélation de cette mesure laboratoire Cpc avec une mesure de la vitesse de mise à plat du pneumatique $KS_{in}$ obtenue par la mesure du capteur 10 monté sur le pneumatique en particulier pour une profondeur de sol de 10 centimètres. Ainsi, il est aisé de déterminer la classe de résistance du sol sur une profondeur superficielle de 10 centimètres avant le passage du pneumatique à la seule mesure du paramètre $KS_{in}$ et ce quelle que soit la texture, la structure et l'état hydrique du sol ainsi que les conditions d'usage du pneumatique que sont la pression de gonflage, la charge appliquée et la vitesse de roulage.

Ainsi, en notant F le facteur de propriété mécanique sol associé à la résistance du sol devant le pneu noté $Cpc_{in}$, et f une fonction correspondant à la relation et portant sur le paramètre $KS_{in}$, on peut écrire :

[Math 1]

$$F = f(KS_{in})$$

Plus précisément, la relation linéaire peut être de la forme :

[Math 2]

$$F = a + b * KS_{in}$$

avec F le facteur de résistance mécanique du sol devant le pneu sur une profondeur de X centimètres, $KS_{in}$ la vitesse de mise à plat du pneumatique et a, b des coefficients réels non nuls fixes préalablement déterminés.

Les coefficients fixes a, b sont de préférence choisis afin de maximiser la discrimination de classes de propriété mécaniques du sol. On peut par exemple utiliser une analyse discriminante à une dimension. Cette analyse discriminante vise à maximiser les écarts entre les centres de gravité de chacune des classes de propriété mécanique du sol, tout en minimisant la dispersion intra-classe.

A titre d'exemple illustratif et non limitatif, le facteur de résistance mécanique du sol devant le pneu sur une profondeur de 10 centimètres peut être déterminé de la façon suivante :

[Math 3]

$$Cpc_{in}\ 0\text{-}10\ (bar) = -0.092 - 1.109 \times KS_{in}$$

Les lignes en trait pointillé autour de la ligne en trait continu qui illustre la formule ci-dessus, délimité l'intervalle de confiance à 90 %.

L'illustration de la Fig. 4b concerne une analyse similaire qui a été menée sur les mêmes sols en termes de structure, de texture et d'état hydrique que ceux de la Fig. 4a.

La seconde propriété mécanique observée est la résistance du sol derrière le pneumatique $Cpc_{out}$ qui correspond à la détermination de la limité de contrainte élastique admissible par le sol, exprimé en bar, après le passage du pneumatique. Si le sol est resté élastique, on n'observe aucune variation de la résistance du sol devant ou derrière le pneumatique, c'est à-dire pas de formation d'ornière en général. Généralement, sur un sol agraire, on observe un tassement du sol par le passage du pneumatique que l'on essaye de minimiser en modifiant pas exemple la pression de gonflage du pneumatique agricole. De ce fait, le $Cpc_{out}$ est généralement au-dessus du $Cpc_{in}$ ce qui traduit le tassement ou déformation plastique subie par le sol en raison de l'action mécanique exercée par le pneumatique sur le sol.

Il est possible de définir des classes de résistance du sol en particulier trois niveaux différents, ce qui permet de différencier la nature de résistance du sol à l'aide d'une caractérisation de type laboratoire de la résistance du sol obtenue par une mesure de compression uni-axiale d'un échantillon cylindrique de terre prélevé à une certaine profondeur X. Les mêmes mesures de Cpc ont été employées que pour la Fig. 4a.

A titre d'exemple illustratif et non limitatif, les classes suivantes peuvent être utilisées :

[Table 2]

| Cpc (bar) | <= 0.5 bar | 0.5 - 1.0 bar | > 1.0 bar |
|---|---|---|---|
| Résistance du sol | Faible ou « lâche » | Moyenne ou « intermédiaire » | Forte ou « résistant » |
| $KS_{out}$ | <= 0.8 | 0.8 - 1.6 | > 1.6 |

Il a été identifié un niveau de corrélation similaire entre la mesure laboratoire de compression uni-axiale Cpc, en particulier pour une profondeur de sol de 10 centimètres, et la détermination de la vitesse de mise en rond du pneumatique $KS_{out}$ comme l'illustre la Fig. 4b.

Il est donc aisé de déterminer la classe de résistance du sol $Cpc_{out}$ sur une profondeur superficielle de 10 centimètres avant le passage du pneumatique à la seule mesure du paramètre $KS_{out}$ et ce quelle que soit la texture, la structure et l'état hydrique du sol ainsi que les conditions d'usage du pneumatique que sont la pression de gonflage, la charge appliquée et la vitesse de roulage.

Ainsi, en notant F le facteur de propriété mécanique sol associé à la résistance du sol derrière le pneu noté $Cpc_{out}$, et f une fonction correspondant à la relation et portant sur le paramètre $KS_{out}$, on peut écrire :

[Math 4]

$$F = f(KS_{out})$$

Plus précisément, la relation linéaire peut être de la forme :

[Math 5]

$$F = a + b * KS_{out}$$

avec F le facteur de résistance mécanique du sol derrière le pneu, $KS_{out}$ la vitesse de mise en rond du pneumatique et a, b des coefficients réels non nuls fixes préalablement déterminés.

Les coefficients fixes a, b sont de préférence choisis afin de maximiser la discrimination de classes de propriété mécaniques du sol. On peut par exemple utiliser une analyse discriminante à une dimension. Cette analyse discriminante vise à maximiser les écarts entre les centres de gravité de chacune des classes de propriété mécanique du sol, tout en minimisant la dispersion intra-classe.

A titre d'exemple illustratif et non limitatif, le facteur de résistance mécanique du sol derrière le pneu sur une profondeur de 10 centimètres peut être déterminé de la façon suivante :

[Math 6]

$$Cpc_{out} \ 0\text{-}10 \ (bar) = 0.142 + 0.507 \ x \ KS_{out}$$

Les lignes en trait pointillé autour de la ligne en trait continu qui illustre la formule ci-dessus, délimité l'intervalle de confiance à 90 %.

Les valeurs de vitesse de mise à plat ou de mise en rond du pneumatique pour la même classe de sol est différent du fait que l'interaction entre le sol et le pneumatique est différente que l'on se situe à l'entrée ou la sortie de l'aire de contact ce qui justifie des relations spécifiques pour chaque grandeur.

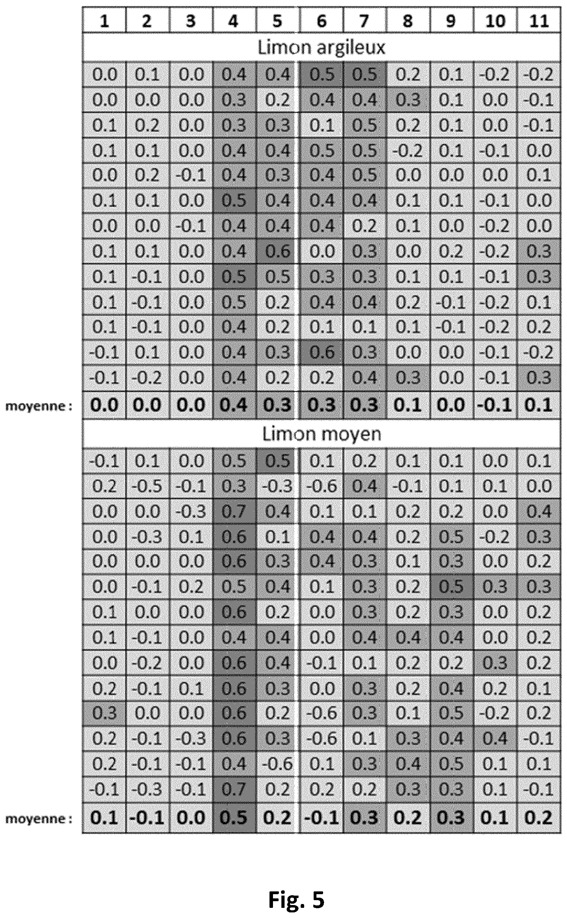

La Fig. 5 est une cartographie de la compaction superficielle du sol $C_{pneu}$ engendrée par le passage du pneumatique à l'échelle de deux parcelles agricoles se différentiant par une texture différente mais toutes deux fortement humides sur toute la profondeur du sol (condition hivernale).

La compaction superficielle du sol $C_{pneu}$ engendrée par le passage du pneumatique est évaluée en comparant les résistances mécaniques du sol devant, $Cpc_{in}$, et derrière, $Cpc_{out}$, le pneumatique. Dans notre cas, la comparaison consiste à effectuer la différence entre la résistance mécanique du sol après le passage du pneumatique et la résistance mécanique du sol avant le passage du sol. La compaction est alors exprimée en bar. Cependant, la comparaison peut aussi porter sur le rapport entre les deux niveaux de résistance mécanique du sol et la compaction s'exprime alors en pourcentage.

Les colonnes de la cartographie représentent les sillons longitudinaux de passage du pneumatique équipé du capteur 10 de mesure sur la parcelle agricole sous une condition de roulage du pneumatique homogène. Cependant, d'un sillon à l'autre, les conditions d'usage du pneumatique peuvent évoluer en augmentant la charge appliquée au pneumatique ou en modifiant la pression de gonflage du pneumatique par exemple. Dans l'exemple donné, on considère ici que toute la parcelle présente les mêmes propriétés physiques en termes de texture et de structure initiale. Cependant, certains sillons ont subi un travail de préparation spécifique qui rend la structure de ce sillon différent des autres sillons potentiellement. Certains sillons de la même parcelle sont dans une structure de type WO, ou W10 ou W30 et ont subi jusqu'à trois passages de tracteur.

Pour le même sillon, c'est à dire la même colonne pour une parcelle donnée, les diverses lignes correspondent à des unités linéiques du sillon correspondant à un tour de roue du pneumatique de mesure soit environ 5 mètres de distance. Pour chaque tour de roue, on mesure les vitesses de mise à plat $KS_{in}$ et de mise en rond $KS_{out}$ du pneumatique. A partir de ces valeurs on en déduit un $Cpc_{in}$ et un $Cpc_{out}$ sur la base des formules précédentes. On évalue alors la compaction superficielle du sol $C_{pneu}$ à chaque tour de roue.. Enfin, on moyenne l'ensemble des valeurs de compaction superficielle du sol de chaque unité linéique de sillon sur l'ensemble des unités linéiques du même sillon.

On observe que certains sillons ne détectent aucune compaction superficielle du sol comme les premières colonnes situées à gauche, ce qui traduit que le passage du pneumatique ne modifie pas la structure du sol. C'est à-dire que le sol reste dans un état élastique à la suite du passage du pneumatique dans les conditions d'utilisation du pneumatique sur ces sillons.

En revanche d'autres sillons correspondant aux sillons 4 à 7 depuis la gauche présentent de fort taux de compaction du sol engendré par le pneumatique, de l'ordre de 0,3 bars en général, qui traduit une forte déformation plastique du sol par le passage du pneumatique et conduit à la formation d'ornière. Il convient alors pour éviter la formation d'ornière d'adapter les conditions d'usage du pneumatique en réduisant sa pression de gonflage ou en diminuant sa charge transportée. Cependant si le sol est lâche dans ce sillon, ce qui sera détecté par la relation liant le $Cpc_{in}$ à la vitesse de mise à plat du pneumatique, ce tassement peut être désiré. Il convient d'adapter le comportement en fonction de la fonction souhaitée par rapport à la fonction obtenue.

Une telle cartographie montre un potentiel agraire évident en analysant l'hétérogénéité potentielle du sol d'une parcelle agricole ce qui permet d'adapter à priori les conditions d'usage du pneumatique à chaque zone de la parcelle considérée. De plus, la mesure en temps réel des mesures et de la cartographie permet d'adapter aussi en temps réel les conditions d'utilisation du pneumatique au cours de travail de la parcelle pour minimiser l'impact de l'engin agricole sur la propriété mécaniques du sol. Par exemple, ces mesures peuvent servir à alerter d'un risque de formation d'ornière nuisible à l'exploitation de la parcelle et d'adapter au plus vite les conditions d'utilisation du pneumatique aux propriétés mécaniques du sol à l'échelle d'une unité linéique de sillon.

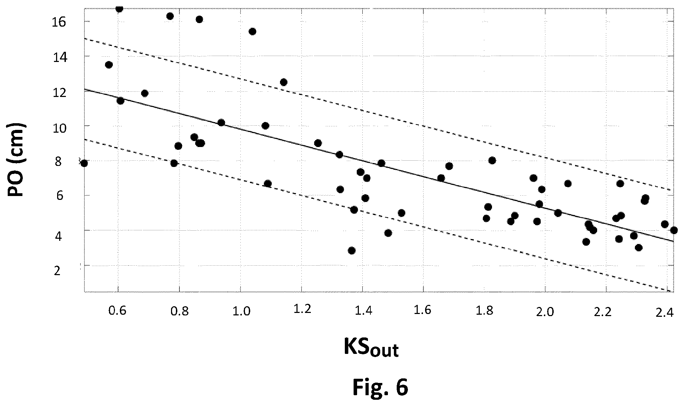

L'illustration de la Fig. 6 concerne une analyse similaire qui a été menée sur les mêmes sols en termes de structure, de texture et d'état hydrique que ceux des Fig. 4a et Fig. 4b. Mais cette fois-ci la propriété mécanique est globale à l'échelle du pneumatique.

La propriété mécanique observée est la profondeur d'ornière PO qui correspond à la formation d'une ornière dans le sol agraire, exprimé en centimètre, en raison de la déformation plastique subie par sol après le passage du pneumatique. Le fait que c'est la résultante du passage complet du pneumatique sur le sol justifie le caractère global de cette propriété mécanique du sol. Si le sol est resté élastique, on n'observe aucune déformation plastique du sol, c'est à-dire pas de formation d'ornière. Généralement, sur un sol agraire de faible résistance par nature, on observe un tassement du sol par le passage du pneumatique que l'on essaye de minimiser en modifiant par exemple la pression de gonflage du pneumatique agricole. De ce fait, l'action mécanique exercée par le pneumatique sur le sol génère dans le sol une ornière de profondeur plus ou moins élevée.

Il a été identifié un niveau de corrélation entre la mesure de la profondeur d'ornière après le passage du pneumatique et les vitesses de mise à plat $KS_{in}$ et de mise en rond $KS_{ou}$ du pneumatique. Ceci est à lier à la variation de la limite de plasticité du sol lors du passage du pneumatique et qui se traduit par une variation des résistances du sol devant, $Cpc_{in}$, et derrière, $Cpc_{out}$, le pneumatique sur une profondeur X du sol.

Cependant, la profondeur du sol X à prendre en compte pour corréler au mieux avec la profondeur d'ornière dépend fortement des propriétés physiques du sol.

Il a été identifié un niveau de corrélation générale et globale entre la profondeur d'ornière PO et la vitesse de mise en rond $KS_{out}$ du pneumatique au travers d'une relation simple entre les deux grandeurs.

En effet, la corrélation lie cette mesure de terrain, à postériori du passage du pneumatique avec une mesure de la vitesse de mise en rond du pneumatique $KS_{out}$ obtenue par la mesure du capteur 10 monté sur le pneumatique en particulier pour une profondeur de sol de 10 centimètres. Ainsi, il est aisé de déterminer la profondeur d'ornière du sol après le passage du pneumatique à la seule mesure du paramètre $KS_{out}$ et ce quelle que soit la texture, la structure et l'état hydrique du sol et les conditions d'usage du pneumatique que sont la pression de gonflage, la charge appliquée et la vitesse de roulage.

Ainsi, en notant F le facteur de propriété mécanique du sol associé à profondeur d'ornière PO et f une fonction correspondant à la relation et portant sur le paramètre $KS_{out}$, on peut écrire :

[Math 7a]

$$F = f(KS_{out})$$

,

ou

[Math 7b]

$$F = f(KS_{out}, KS_{in})$$

Plus précisément, la relation linéaire peut être aussi de la forme :

[Math 8a]

$$F = a + b * KS_{out}$$

,

ou

[Math 8b]

$$F = a + b * KS_{out} + c * KS_{in}$$

avec F le facteur de profondeur d'ornière, $KS_{out}$ la vitesse de mise en rond du pneumatique, $KS_{in}$ la vitesse de mise à plat du pneumatique et a, b, c des coefficients réels non nuls fixes préalablement déterminés.

A titre d'exemple illustratif et non limitatif, le facteur de profondeur d'ornière PO peut être déterminé à partir de la vitesse de mise en rond $KS_{out}$ du pneumatique de la façon suivante :

[Math 9]

$$PO\ (cm) = 14.3 - 4.5 \times KS_{out}$$

Les lignes en trait pointillé autour de la ligne en trait continu qui illustre la formule ci-dessus, délimité l'intervalle de confiance à 90 %.

## Revendications

1. Procédé de détermination des propriétés mécaniques d'un sol sur lequel roule un pneumatique (1) monté sur un véhicule (9), ledit pneumatique (1) étant muni d'un capteur (10) configuré pour acquérir un signal de mesure représentatif de l'évolution de la courbure du pneumatique lors du roulement sur un sol (7), le procédé comprenant les étapes suivantes :

- acquisition (S1) par le capteur (10) d'un signal de mesure représentatif de l'évolution de la courbure du pneumatique (1) au cours du roulement,
- détermination (S2) à partir du signal de mesure de données de mesure comprenant :

a) un premier paramètre ($KS_{in}$) représentatif d'une vitesse, préférentiellement angulaire, de mise à plat du pneumatique (1) lors du contact avec le sol (7) au cours d'un tour de roue du pneumatique, et
b) un deuxième paramètre ($KS_{out}$) représentatif d'une vitesse, préférentiellement angulaire, de mise en rond du pneumatique (1) lors du décollement avec le sol (7) au cours d'un tour de roue du pneumatique,

- détermination (S4) de la propriété mécanique du sol en fonction du premier paramètre ($KS_{in}$) et/ou du deuxième paramètre ($KS_{out}$) en utilisant une relation linéaire liant ladite propriété mécanique du sol et le premier paramètre ($KS_{in}$) et/ou le deuxième paramètre ($KS_{out}$), la propriété mécanique du sol étant compris dans le groupe comprenant la profondeur d'ornière (PO), la résistance mécanique du sol à l'entrée de l'aire de contact ($Cpc_{in}$), la résistance mécanique du sol à la sortie de l'aire de contact ($Cpc_{out}$), la compaction du sol engendré par le pneumatique ($C_{pneu}$).

2. Procédé selon la revendication 1, dans lequel, lors du roulement, au cours d'un tour de roue, la courbure du pneumatique (1) évolue selon un cycle présentant :

- une partie hors contact avec le sol,
- une partie en contact avec le sol, dans lequel le premier paramètre ($KS_{in}$) est déterminé à partir d'une partie du signal de mesure correspondant à une transition de la courbure du pneumatique (1) entre la partie hors contact avec le sol et la partie en contact avec le sol, et le deuxième paramètre ($KS_{out}$) est déterminé à partir d'une partie du signal de mesure correspondant à une transition de la courbure du pneumatique (1) entre la partie en contact avec le sol et la partie hors contact avec le sol.

3. Procédé selon la revendication 1 ou 2, dans lequel, lors du roulement, au cours d'un tour de roue, la courbure du pneumatique (1) évolue selon un cycle présentant :

- une partie hors contact avec le sol se caractérisant sur le signal de mesure par une courbure stable,
- une partie en contact avec le sol se caractérisant sur le signal de mesure par un pic de variation de courbure de contact (20, 30),
- une transition dite transition d'entrée entre la partie hors contact avec le sol et la partie en contact avec le sol, se caractérisant sur le signal de mesure par un pic de variation de courbure d'entrée (21, 22) opposé au pic de variation de courbure de contact (20, 30),
- une transition dite transition de sortie entre la partie en contact avec le sol et la partie hors contact avec le sol, se caractérisant sur le signal de mesure par un pic de variation de courbure de sortie (22, 32) opposé au pic de variation de courbure de contact (20, 30), le premier paramètre ($KS_{in}$) étant déterminé par une pente entre le pic de variation de courbure d'entrée (21) et le pic de variation de courbure de contact (20), le second paramètre ($KS_{out}$) étant déterminé par une pente entre le pic de variation de courbure d'entrée (31) et le pic de variation de courbure de contact (30).

4. Procédé selon l'une des revendications précédentes, dans lequel la relation linéaire est de la forme :

$$F = a + b \times KS_{i\ ou\ j},$$

ou

$$F = a + b \times KS_i + c \times KS_j$$

avec F un facteur de propriété mécanique, $KS_{i\ ou\ j}$ le premier ou le deuxième paramètre, et a, b, c des coefficients fixes préalablement déterminés.

5. Procédé selon l'une des revendications précédentes, dans lequel la propriété mécanique du sol est déterminée en calculant un facteur de propriété mécanique (F) à partir du premier paramètre ($KS_{in}$) et/ou du second paramètre ($KS_{out}$), et en comparant ledit facteur de propriété mécanique à des seuils délimitant des classes de propriété mécanique du sol.

6. Procédé selon l'une des revendications précédentes, dans lequel la propriété mécanique du sol est définie sur une profondeur X de sol inférieure à 40 centimètres, potentiellement inférieure 20 centimètres, voire inférieure à 10 centimètres.

**7.** Procédé selon l'une des revendications précédentes comprenant une étape de localisation du véhicule (9) au cours de l'étape d'acquisition du signal délivrant une position au moins bidimensionnelle (Ploc) du véhicule (9).

**8.** Unité de traitement de données (15) configurée pour recevoir des données de mesure dérivées d'un signal de mesure représentatif de l'évolution de la courbure du pneumatique lors du roulement sur un sol, lesdites données de mesure comprenant :

   a) un premier paramètre ($KS_{in}$) représentatif d'une vitesse, préférentiellement angulaire, de mise à plat du pneumatique (1) lors du contact avec le sol (7) au cours d'un tour de roue du pneumatique, et
   b) un deuxième paramètre ($KS_{out}$) représentatif d'une vitesse, préférentiellement angulaire, de mise en rond du pneumatique (1) lors du décollement avec le sol (7) au cours d'un tour de roue du pneumatique, l'unité de traitement de données (15) étant configurée pour déterminer la propriété mécanique du sol en fonction du premier paramètre ($KS_{in}$) et/ou du deuxième paramètre ($KS_{out}$) en utilisant une relation linéaire liant ladite propriété mécanique du sol et le premier paramètre ($KS_{in}$) et/ou le deuxième paramètre ($KS_{out}$), la propriété mécanique du sol étant compris dans le groupe comprenant la profondeur d'ornière (PO), la résistance mécanique du sol à l'entrée de l'aire de contact ($Cpc_{in}$), la résistance mécanique du sol à la sortie de l'aire de contact ($Cpc_{out}$), la compaction du sol engendré par le pneumatique ($C_{pneu}$), préférentiellement l'unité de traitement de données (15) est configurée pour recevoir la position au moins bidimensionnelle (Ploc) du véhicule (9) associée aux données de mesure.

**9.** Véhicule (9) comprenant :

   - au moins un pneumatique (1),
   - au moins capteur (10) sensible à l'évolution de la courbure du pneumatique, configuré pour générer un signal de mesure représentatif de l'évolution de la courbure du pneumatique lors du roulement sur un sol,
   - une unité de traitement de données (15) selon la revendication 8, le véhicule étant configuré pour mettre en œuvre le procédé selon l'une quelconque des revendications 1 à 6.

**10.** Véhicule (9) selon la revendication précédente, dans lequel le capteur (10) est disposé à l'intérieur du pneumatique (1).

**11.** Véhicule (9) selon la revendication précédente, dans lequel le capteur (10) comprend une partie active (11) et une carte électronique (12), la partie active (11) étant configurée pour générer le signal de mesure, la carte électronique (12) étant configurée pour déterminer les données de mesure, et dans lequel l'unité de traitement de données (15) est disposée à l'extérieur du pneumatique.

**Patentansprüche**

**1.** Verfahren zur Bestimmung der mechanischen Eigenschaften eines Bodens, auf dem ein an einem Fahrzeug (9) montierter Reifen (1) rollt, wobei der Reifen (1) mit einem Sensor (10) versehen ist, der dazu ausgestaltet ist, ein Messsignal zu erfassen, das für den Verlauf der Krümmung des Reifens beim Rollen auf einem Boden (7) repräsentativ ist, wobei das Verfahren die folgenden Schritte umfasst:

   - Erfassen (S1) eines Messsignals, das für den Verlauf der Krümmung des Reifens (1) beim Rollen repräsentativ ist, durch den Sensor (10),
   - Bestimmen (S2), ausgehend von dem Messsignal, von Messdaten, umfassend:

      a) einen ersten Parameter ($KS_{in}$), der für eine Geschwindigkeit, vorzugsweise eine Winkelgeschwindigkeit, der Abplattung des Reifens (1) beim Kontakt mit dem Boden (7) während einer Radumdrehung des Reifens repräsentativ ist, und
      b) einen zweiten Parameter ($KS_{out}$), der für eine Geschwindigkeit, vorzugsweise eine Winkelgeschwindigkeit, der Rückverformung des Reifens (1) beim Lösen von dem Boden (7) während einer Radumdrehung des Reifens repräsentativ ist,

   - Bestimmen (S4) der mechanischen Eigenschaft des Bodens in Abhängigkeit von dem ersten Parameter ($KS_{in}$)

und/oder dem zweiten Parameter ($KS_{out}$) unter Verwendung einer linearen Beziehung, die die mechanische Eigenschaft des Bodens und den ersten Parameter ($KS_{in}$) und/oder den zweiten Parameter ($KS_{out}$) verknüpft, wobei die mechanische Eigenschaft des Bodens in der Gruppe enthalten ist, die die Spurrinnentiefe (PO), die mechanische Festigkeit des Bodens beim Eintreten in die Kontaktfläche ($Cpc_{in}$), die mechanische Festigkeit des Bodens beim Austreten aus der Kontaktfläche ($Cpc_{out}$), die durch den Reifen bewirkte Verdichtung des Bodens ($C_{Reifen}$) umfasst.

2. Verfahren nach Anspruch 1, wobei die Krümmung des Reifens (1) beim Rollen während einer Radumdrehung nach einem Zyklus verläuft, der Folgendes aufweist:

- einen Teil ohne Bodenkontakt,
- einen Teil mit Bodenkontakt, wobei der erste Parameter ($KS_{in}$) ausgehend von einem Teil des Messsignals bestimmt wird, der einem Übergang der Krümmung des Reifens (1) zwischen dem Teil ohne Bodenkontakt und dem Teil mit Bodenkontakt entspricht, und der zweite Parameter ($KS_{out}$) ausgehend von einem Teil des Messsignals bestimmt wird, der einem Übergang der Krümmung des Reifens (1) zwischen dem Teil mit Bodenkontakt und dem Teil ohne Bodenkontakt entspricht.

3. Verfahren nach Anspruch 1 oder 2, wobei die Krümmung des Reifens (1) beim Rollen während einer Radumdrehung nach einem Zyklus verläuft, der Folgendes aufweist:

- einen Teil ohne Bodenkontakt, der an dem Messsignal durch eine gleich bleibende Krümmung gekennzeichnet ist,
- einen Teil mit Bodenkontakt, der an dem Messsignal durch einen Kontaktkrümmungsänderungspeak (20, 30) gekennzeichnet ist,
- einen Übergang, Eintrittsübergang genannt, zwischen dem Teil ohne Bodenkontakt und dem Teil mit Bodenkontakt, der an dem Messsignal durch einen Eintrittskrümmungsänderungspeak (21, 22) gekennzeichnet ist, der zu dem Kontaktkrümmungsänderungspeak (20, 30) entgegengesetzt ist,
- einen Übergang, Austrittsübergang genannt, zwischen dem Teil mit Bodenkontakt und dem Teil ohne Bodenkontakt, der an dem Messsignal durch einen Austrittskrümmungsänderungspeak (22, 32) gekennzeichnet ist, der zu dem Kontaktkrümmungsänderungspeak (20, 30) entgegengesetzt ist, wobei der erste Parameter ($KS_{in}$) durch eine Steigung zwischen dem Eintrittskrümmungsänderungspeak (21) und dem Kontaktkrümmungsänderungspeak (20) bestimmt wird, wobei der zweite Parameter ($KS_{out}$) durch eine Steigung zwischen dem Eintrittskrümmungsänderungspeak (31) und dem Kontaktkrümmungsänderungspeak (30) bestimmt wird.

4. Verfahren nach einem der vorhergehenden Ansprüche, wobei die lineare Beziehung die folgende Form hat:

$$F = a + b \times KS_{i \text{ oder } j},$$

oder

$$F = a + b \times KS_i + c \times KS_j$$

wobei F ein Faktor einer mechanischen Eigenschaft ist, $KS_{i \text{ oder } j}$ der erste oder der zweite Parameter ist und a, b, c vorab bestimmte feststehende Koeffizienten sind.

5. Verfahren nach einem der vorhergehenden Ansprüche, wobei die mechanische Eigenschaft des Bodens bestimmt wird, indem ein Faktor (F) einer mechanischen Eigenschaft ausgehend von dem ersten Parameter ($KS_{in}$) und/oder dem zweiten Parameter ($KS_{out}$) berechnet wird und indem der Faktor einer mechanischen Eigenschaft mit Schwellenwerten verglichen wird, die Klassen einer mechanischen Eigenschaft des Bodens begrenzen.

6. Verfahren nach einem der vorhergehenden Ansprüche, wobei die mechanische Eigenschaft des Bodens über eine Bodentiefe X von weniger als 40 cm, potenziell weniger als 20 cm oder sogar weniger als 10 cm definiert ist.

7. Verfahren nach einem der vorhergehenden Ansprüche, umfassend einen Schritt zur Lokalisierung des Fahrzeugs (9) während des Schritts des Erfassens des Signals, der eine mindestens zweidimensionale Position (Ploc) des Fahrzeugs (9) liefert.

8. Datenverarbeitungseinheit (15), die dazu ausgestaltet ist, Messdaten zu empfangen, die aus einem Messsignal abgeleitet sind, das für den Verlauf der Krümmung des Reifens beim Rollen auf einem Boden repräsentativ ist, wobei die Messdaten Folgendes umfassen:

a) einen ersten Parameter ($KS_{in}$), der für eine Geschwindigkeit, vorzugsweise eine Winkelgeschwindigkeit, der Abplattung des Reifens (1) beim Kontakt mit dem Boden (7) während einer Radumdrehung des Reifens repräsentativ ist,
b) einen zweiten Parameter ($KS_{out}$), der für eine Geschwindigkeit, vorzugsweise eine Winkelgeschwindigkeit, der Rückverformung des Reifens (1) beim Lösen von dem Boden (7) während einer Radumdrehung des Reifens repräsentativ ist, wobei die Datenverarbeitungseinheit (15), dazu ausgestaltet ist, die mechanische Eigenschaft des Bodens in Abhängigkeit von dem ersten Parameter ($KS_{in}$) und/oder dem zweiten Parameter ($KS_{out}$) unter Verwendung einer linearen Beziehung zu bestimmen, die die mechanische Eigenschaft des Bodens und den ersten Parameter ($KS_{in}$) und/oder den zweiten Parameter ($KS_{out}$) verknüpft, wobei die mechanische Eigenschaft des Bodens in der Gruppe enthalten ist, die die Spurrinnentiefe (PO), die mechanische Festigkeit des Bodens beim Eintreten in die Kontaktfläche ($Cpc_{in}$), die mechanische Festigkeit des Bodens beim Austreten aus der Kontaktfläche ($Cpc_{out}$), die durch den Reifen bewirkte Verdichtung des Bodens ($C_{Reifen}$) umfasst, wobei die Datenverarbeitungseinheit (15) vorzugsweise dazu ausgestaltet ist, die mindestens zweidimensionale Position (Ploc) des Fahrzeugs (9) zu empfangen, die den Messdaten zugeordnet ist.

9. Fahrzeug (9), umfassend:

- mindestens einen Reifen (1),
- mindestens einen für den Verlauf der Krümmung des Reifens empfindlichen Sensor (10), der dazu ausgestaltet ist, ein Messsignal zu erzeugen, das für den Verlauf der Krümmung des Reifens beim Rollen auf einem Boden repräsentativ ist,
- eine Datenverarbeitungseinheit (15) nach Anspruch 8,

wobei das Fahrzeug dazu ausgestaltet ist, das Verfahren nach einem der Ansprüche 1 bis 6 zu implementieren.

10. Fahrzeug (9) nach dem vorhergehenden Anspruch, wobei der Sensor (10) im Inneren des Reifens (1) angeordnet ist.

11. Fahrzeug (9) nach dem vorhergehenden Anspruch, wobei der Sensor (10) einen aktiven Teil (11) und eine elektronische Platine (12) umfasst, wobei der aktive Teil (11) dazu ausgestaltet ist, das Messsignal zu erzeugen, wobei die elektronische Platine (12) dazu ausgestaltet ist, die Messdaten zu bestimmen, und wobei die Datenverarbeitungseinheit (15) außerhalb des Reifens angeordnet ist.

## Claims

1. Method for determining the mechanical properties of a ground on which a tyre (1) mounted on a vehicle (9) is running, said tyre (1) being fitted with a sensor (10) configured to acquire a measurement signal representative of the change in the curvature of the tyre as it runs over a ground (7), the method comprising the following steps:

- acquiring (S1), by way of the sensor (10), a measurement signal representative of the change in the curvature of the tyre (1) while it is running,
- determining (S2), from the measurement signal, measurement data comprising:

a) a first parameter ($KS_{in}$) representative of a rate, preferably angular rate, at which the tyre (1) flattens on contact with the ground (7) over the course of one revolution of the wheel bearing the tyre, and
b) a second parameter ($KS_{out}$) representative of a rate, preferably angular rate, at which the tyre (1) regains its shape on becoming separated from the ground (7) over the course of one revolution of the wheel bearing the tyre,

- determining (S4) the mechanical property of the ground as a function of the first parameter ($KS_{in}$) and/or the second parameter ($KS_{out}$) using a linear relationship connecting said mechanical property of the ground and the first parameter ($KS_{in}$) and/or the second parameter ($KS_{out}$) the mechanical property of the ground being included in the group comprising the rut depth (RD), the mechanical resistance of the ground on entering the contact patch ($Cpc_{in}$), the mechanical resistance of the ground on leaving the contact patch ($Cpc_{out}$), and the compaction of the ground caused by the tyre ($C_{tyre}$),

2. Method according to Claim 1, wherein, during running, over the course of one revolution of the wheel, the curvature of the tyre (1) changes according to a cycle exhibiting:

 - a part where there is no contact with the ground,
 - a part where there is contact with the ground, wherein the first parameter ($KS_{in}$) is determined from part of the measurement signal corresponding to a transition in the curvature of the tyre (1) between the part where there is no contact with the ground and the part where there is contact with the ground, and the second parameter ($KS_{out}$) is determined from part of the measurement signal corresponding to a transition in the curvature of the tyre (1) between the part where there is contact with the ground and the part where there is no contact with the ground.

3. Method according to Claim 1 or 2, wherein, during running, over the course of one revolution of the wheel, the curvature of the tyre (1) changes according to a cycle exhibiting:

 - a part where there is no contact with the ground, **characterized in** the measurement signal by a stable curvature,
 - a part where there is contact with the ground, **characterized in** the measurement signal by a contact curvature variation spike (20, 30),
 - a transition referred to as the coming-into-contact transition between the part where there is no contact with the ground and the part where there is contact with the ground, **characterized in** the measurement signal by a coming-into-contact curvature variation spike (21, 22) that is the opposite of the contact curvature variation spike (20, 30),
 - a transition referred to as the coming-out-of-contact transition between the part where there is contact with the ground and the part where there is no contact with the ground, **characterized in** the measurement signal by a coming-out-of-contact curvature variation spike (22, 32) that is the opposite of the contact curvature variation spike (20, 30), the first parameter ($KS_{in}$) being determined by a gradient between the coming-into-contact curvature variation spike (21) and the contact curvature variation spike (20), the second parameter ($KS_{out}$) being determined by a gradient between the coming-into-contact curvature variation spike (31) and the contact curvature variation spike (30).

4. Method according to one of the preceding claims, wherein the linear relationship takes the following form:

$$F = a + b \times KS_{i\ or\ j},$$

 or

$$F = a + b \times KS_i + c \times KS_j$$

 where F is a mechanical property factor, $KS_{i\ or\ j}$ is the first or the second parameter, and a, b and c are predetermined fixed coefficients.

5. Method according to one of the preceding claims, wherein the mechanical property of the ground is determined by calculating a mechanical property factor (F) from the first parameter ($KS_{in}$) and/or the second parameter ($KS_{out}$), and by comparing said mechanical property factor to thresholds delimiting mechanical property categories for the ground.

6. Method according to one of the preceding claims, wherein the mechanical property of the ground is defined over a ground depth X of less than 40 centimetres, potentially less than 20 centimetres, or even less than 10 centimetres.

7. Method according to one of the preceding claims, comprising a step of locating the vehicle (9) during the step of acquiring the signal that provides an at least two-dimensional position (Ploc) of the vehicle (9).

8. Data processing unit (15) configured to receive measurement data derived from a measurement signal representative of the change in the curvature of the tyre as it runs over a ground, said measurement data comprising:

 a) a first parameter ($KS_{in}$) representative of a rate, preferably angular rate, at which the tyre (1) flattens on contact with the ground (7) over the course of one revolution of the wheel bearing the tyre, and

b) a second parameter ($KS_{out}$) representative of a rate, preferably angular rate, at which the tyre (1) regains its shape on becoming separated from the ground (7) over the course of one revolution of the wheel bearing the tyre, the data processing unit (15) being configured to determine the mechanical property of the ground as a function of the first parameter ($KS_{in}$) and/or the second parameter ($KS_{out}$) using a linear relationship connecting said mechanical property of the ground and the first parameter ($KS_{in}$) and/or the second parameter ($KS_{out}$) the mechanical property of the ground being included in the group comprising the rut depth (RD), the mechanical resistance of the ground on entering the contact patch ($Cpc_{in}$), the mechanical resistance of the ground on leaving the contact patch ($Cpc_{out}$), and the compaction of the ground caused by the tyre ($C_{tyre}$)., and the data processing unit (15) is preferably configured to receive that at least two-dimensional position (Ploc) of the vehicle (9) that is associated with the measurement data.

9. Vehicle (9) comprising:

- at least one tyre (1),
- at least one sensor (10) sensitive to the change in the curvature of the tyre and configured to generate a measurement signal representative of the change in the curvature of the tyre as it runs over a ground,
- a data processing unit (15) according to Claim 8,

the vehicle being configured to implement the method according to any one of Claims 1 to 6.

10. Vehicle (9) according to the preceding claim, wherein the sensor (10) is disposed inside the tyre (1).

11. Vehicle (9) according to the preceding claim, wherein the sensor (10) comprises an active part (11) and an electronic circuit board (12), the active part (11) being configured to generate the measurement signal, the electronic circuit board (12) being configured to determine the measurement data, and wherein the data processing unit (15) is disposed outside the tyre.

**Fig. 1**

**Fig. 2**

**Fig. 3**

**Fig. 4a**

**Fig. 4b**

| 1 | 2 | 3 | 4 | 5 | 6 | 7 | 8 | 9 | 10 | 11 |
|---|---|---|---|---|---|---|---|---|---|---|
| Limon argileux | | | | | | | | | | |
| 0.0 | 0.1 | 0.0 | 0.4 | 0.4 | 0.5 | 0.5 | 0.2 | 0.1 | -0.2 | -0.2 |
| 0.0 | 0.0 | 0.0 | 0.3 | 0.2 | 0.4 | 0.4 | 0.3 | 0.1 | 0.0 | -0.1 |
| 0.1 | 0.2 | 0.0 | 0.3 | 0.3 | 0.1 | 0.5 | 0.2 | 0.1 | 0.0 | -0.1 |
| 0.1 | 0.1 | 0.0 | 0.4 | 0.4 | 0.5 | 0.5 | -0.2 | 0.1 | -0.1 | 0.0 |
| 0.0 | 0.2 | -0.1 | 0.4 | 0.3 | 0.4 | 0.5 | 0.0 | 0.0 | 0.0 | 0.1 |
| 0.1 | 0.1 | 0.0 | 0.5 | 0.4 | 0.4 | 0.4 | 0.1 | 0.1 | -0.1 | 0.0 |
| 0.0 | 0.0 | -0.1 | 0.4 | 0.4 | 0.4 | 0.2 | 0.1 | 0.0 | -0.2 | 0.0 |
| 0.1 | 0.1 | 0.0 | 0.4 | 0.6 | 0.0 | 0.3 | 0.0 | 0.2 | -0.2 | 0.3 |
| 0.1 | -0.1 | 0.0 | 0.5 | 0.5 | 0.3 | 0.3 | 0.1 | 0.1 | -0.1 | 0.3 |
| 0.1 | -0.1 | 0.0 | 0.5 | 0.2 | 0.4 | 0.4 | 0.2 | -0.1 | -0.2 | 0.1 |
| 0.1 | -0.1 | 0.0 | 0.4 | 0.2 | 0.1 | 0.1 | 0.1 | -0.1 | -0.2 | 0.2 |
| -0.1 | 0.1 | 0.0 | 0.4 | 0.3 | 0.6 | 0.3 | 0.0 | 0.0 | -0.1 | -0.2 |
| -0.1 | -0.2 | 0.0 | 0.4 | 0.2 | 0.2 | 0.4 | 0.3 | 0.0 | -0.1 | 0.3 |
| moyenne : 0.0 | 0.0 | 0.0 | 0.4 | 0.3 | 0.3 | 0.3 | 0.1 | 0.0 | -0.1 | 0.1 |
| Limon moyen | | | | | | | | | | |
| -0.1 | 0.1 | 0.0 | 0.5 | 0.5 | 0.1 | 0.2 | 0.1 | 0.1 | 0.0 | 0.1 |
| 0.2 | -0.5 | -0.1 | 0.3 | -0.3 | -0.6 | 0.4 | -0.1 | 0.1 | 0.1 | 0.0 |
| 0.0 | 0.0 | -0.3 | 0.7 | 0.4 | 0.1 | 0.1 | 0.2 | 0.2 | 0.0 | 0.4 |
| 0.0 | -0.3 | 0.1 | 0.6 | 0.1 | 0.4 | 0.4 | 0.2 | 0.5 | -0.2 | 0.3 |
| 0.0 | 0.0 | 0.0 | 0.6 | 0.3 | 0.4 | 0.3 | 0.1 | 0.3 | 0.0 | 0.2 |
| 0.0 | -0.1 | 0.2 | 0.5 | 0.4 | 0.1 | 0.3 | 0.2 | 0.5 | 0.3 | 0.3 |
| 0.1 | 0.0 | 0.0 | 0.6 | 0.2 | 0.0 | 0.3 | 0.2 | 0.3 | 0.0 | 0.2 |
| 0.1 | -0.1 | 0.0 | 0.4 | 0.4 | 0.0 | 0.4 | 0.4 | 0.4 | 0.0 | 0.2 |
| 0.0 | -0.2 | 0.0 | 0.6 | 0.4 | -0.1 | 0.1 | 0.2 | 0.2 | 0.3 | 0.2 |
| 0.2 | -0.1 | 0.1 | 0.6 | 0.3 | 0.0 | 0.3 | 0.2 | 0.4 | 0.2 | 0.1 |
| 0.3 | 0.0 | 0.0 | 0.6 | 0.2 | -0.6 | 0.3 | 0.1 | 0.5 | -0.2 | 0.2 |
| 0.2 | -0.1 | -0.3 | 0.6 | 0.3 | -0.6 | 0.1 | 0.3 | 0.4 | 0.4 | -0.1 |
| 0.2 | -0.1 | -0.1 | 0.4 | -0.6 | 0.1 | 0.3 | 0.4 | 0.5 | 0.1 | 0.1 |
| -0.1 | -0.3 | -0.1 | 0.7 | 0.2 | 0.2 | 0.2 | 0.3 | 0.3 | 0.1 | -0.1 |
| moyenne : 0.1 | -0.1 | 0.0 | 0.5 | 0.2 | -0.1 | 0.3 | 0.2 | 0.3 | 0.1 | 0.2 |

**Fig. 5**

**Fig. 6**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 3088249 A3